# EUROPEAN PATENT APPLICATION

(11) **EP 3 244 190 A1**
(43) Date of publication of application: **15.11.2017**
(21) Application number: 15876944.8
(22) Date of filing: 19.10.2015
(51) Int. Cl.: G01N 3/04, A61L 27/00

(54) **FIXTURE FOR BIOLOGICAL TISSUE AND METHOD FOR ATTACHING SAME**

(30) Priority: 08.01.2015 JP 2015002681
(71) Applicant: Waseda University, Tokyo 169-8050 (JP)
(72) Inventor: IWASAKI, Kiyotaka, Tokyo 169-8050 (JP); SAITO, Shumpei, Tokyo 169-8050 (JP)
(74) Representative: Grünecker Patent- und Rechtsanwälte PartG mbB
(86) International application number: PCT/JP2015/079444
(87) International publication number: WO 2016/111071

(57) **Abstract**

When a tensile testing machine exerts tensile force to biological tissue attached to a bone, twist and inclination of the biological tissue are so adjusted that the biological tissue and the bone can be integrally set on the tensile testing machine. A fixture 10 according to the present invention includes a holder 11, which holds a bone B, and a support 12, which supports the holder 11. The holder 11 is provided with around-bone-axis angle adjusting mechanisms 14, 15, which adjust the angle of rotation of the bone B around the bone axis of the bone B held by the holder 11. The holder 11 and the support 12 are provided with bone inclination angle adjusting mechanisms 15, 34, which adjust the inclination angle of the bone B held by the holder 11. The support 12 is provided with lateral position adjusting mechanisms 34, 35, which adjust the lateral position of the holder 11 and longitudinal position adjusting mechanisms 35, 36, which adjust the longitudinal position of the holder 11.

## Description

### Technical Field

The present invention relates to a fixture for biological tissue and a method for attaching the same. The present invention particularly relates to a fixture serving as a jig that allows a bone with biological tissue attached thereto to be set on a tensile testing machine for tensile test that is conducted for biological tissue connected to a bone, such as a ligament or a tendon, which tissue, after subjected to animal experimentation, is pulled by the tensile testing machine, and further relates to a method for attaching the fixture.

### Background Art

The anterior cruciate ligament in the knee joint is tissue that links the thigh bone to the shinbone for stable knee joint motion. When the anterior cruciate ligament is damaged, anterior cruciate ligament reconstruction is performed in which a healthy autologous tendon extracted from part of the body of the patient is used to reconstruct the anterior cruciate ligament under the current status that no excellent artificial ligament is available. However, research and development are being conducted for a new artificial ligament which consists of living-body-originated tissue extracted from an animal, intended to allow reconstruction of a damaged anterior cruciate ligament without extraction of an autologous tendon from the patient. In the process of the research and development, dynamic characteristics of artificial ligaments of interest in the research and development have to be evaluated, and it is desirable to prepare a strip-shaped test piece from the artificial ligament before the reconstruction and conduct tensile test or the like for the test piece using a tensile testing machine (see Patent Literature 1, for example).

### Citation List

### Patent Literature

Patent Literature 1: Japanese Patent Laid-Open No. 2013-165740

### Summary of Invention

### Technical Problem

Further, in the process of research and development of a new artificial ligament, after the artificial ligament is transplanted into biological tissue, it is also necessary to evaluate dynamic characteristics of the artificial ligament having undergone joint motion for a fixed period. For example, after the developed artificial ligament is transplanted into the knee joint of an animal, such as a sheep and a pig, it is necessary to force the animal to move for a predetermined period and then evaluate dynamic characteristics of the artificial ligament. Since the reconstructed artificial ligament is linked to the thigh bone and the shinbone with the artificial ligament twisted and further securely adheres to the bones, as in the case of the initial anterior cruciate ligament, it is difficult to take out only the artificial ligament from the knee joint of the animal after the artificial ligament is transplanted into the knee joint and then some period of time elapses. Therefore, to evaluate the dynamic characteristics of the artificial ligament after the reconstruction of the knee joint of an animal, it is inevitable that the animal is killed as a sacrifice, the knee joint is isolated as a whole, and the artificial ligament in the isolated knee joint is tested for the dynamic characteristic evaluation. The artificial ligament linked to the thigh bone and the shinbone is, however, twisted as described above, and when a tensile test is so conducted that the bone axis that is the center line of the thigh bone and the shinbone coincides with the tensile direction, the artificial ligament is pulled with the artificial ligament inclining with respect to the tensile direction or the artificial ligament twisted, resulting in inaccurate comparison in the dynamic characteristic evaluation between the artificial ligament before the reconstruction and the artificial ligament after the reconstruction. Therefore, in this process, the artificial ligament linked to the thigh bone and the shinbone needs to be fixed in the vertical direction, which coincides with the tensile direction, in a constant state, such as a state in which the twist and inclination are eliminated. A jig for fixing an artificial ligament in the manner described above is, however, not currently available.

The present invention has been made to solve the problem described above, and an object of the invention is to provide a biological tissue fixture that is used with a tensile testing machine that exerts tensile force to biological tissue attached to a bone and can adjust twist and inclination of the biological tissue so that the biological tissue and the bone can be integrally set on the tensile testing machine, and another object of the present invention is to provide a method for attaching the fixture.

### Solution to Problem

To achieve the object described above, the present invention relates to a biological tissue fixture that is used when biological tissue connected to a bone is set on a tensile testing machine and fixes the bone with the biological tissue attached thereto, the fixture including a holder that holds the bone and a support that supports the holder. The holder is provided with an around-bone-axis angle adjusting mechanism that adjusts an angle of rotation of the bone around a bone axis of the bone held by the holder. The holder and the support are provided with a bone inclination angle adjusting mechanism that adjusts an inclination angle of the bone held by the holder. The support is provided with a lateral position adjusting mechanism that adjusts a lateral position of the holder and a longitudinal position adjusting mechanism that adjusts a longitudinal position of the holder.

A method for attaching the fixture according to the present invention is a fixture attaching method used when the biological tissue connected to first and second bones is set on the tensile testing machine, the method primarily including fixing the first bone with one fixture described above and the second bone with another fixture described above and attaching one of the fixtures to a fixed side of the tensile testing machine and attaching another of the fixtures to a load cell side of the tensile testing machine.

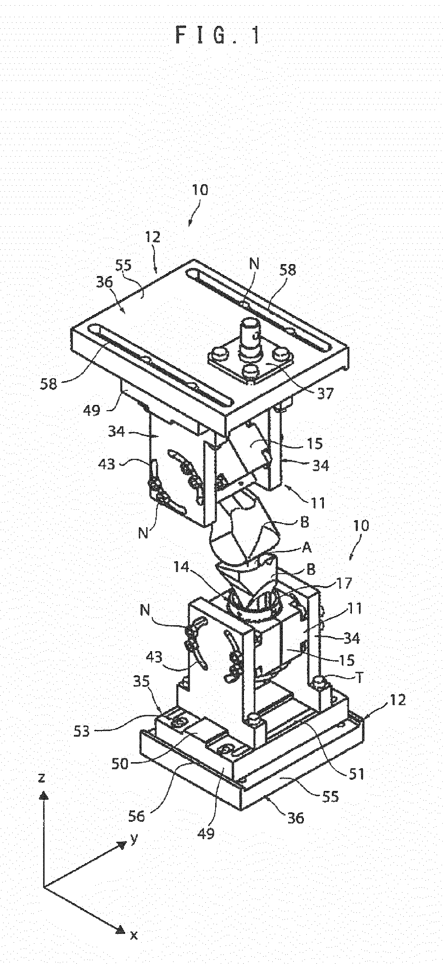

In the claims and the specification of the present invention, the "longitudinal direction" means the direction along the x axis shown in Figure 1, and the "lateral direction" means the direction along the y axis shown in Figure 1 and perpendicular to the x axis in a horizontal plane.

### Advantageous Effects of Invention

According to the present invention, since the posture of a held bone with biological tissue attached thereto can be adjusted in rotational directions around two axes, and the position of the held bone can be adjusted in translational directions along two axes. Therefore, in a case where a tensile testing machine exerts tensile force to the biological tissue attached to the bone, twist and inclination of the biological tissue can be adjusted, and the biological tissue and the bone can be integrally set on the tensile testing machine. That is, the around-bone-axis angle adjusting mechanism can eliminate twist of the biological tissue or adjust the amount of twist of the biological tissue to a prespecified value. Further, adjustment performed by the lateral position adjusting mechanism, the longitudinal position adjusting mechanism, and the bone inclination angle adjusting mechanism allows the biological tissue to be disposed in a posture that agrees with the direction in which the biological tissue is pulled irrespective of the difference in posture of the biological tissue relative to the bone. Moreover, adjustment performed by the bone inclination angle adjusting mechanism allows an inclination angle of the bone with the biological tissue attached thereto to be in a desired state and attached to the tensile testing machine.

### Brief Description of Drawings

[Figure 1] Figure 1 is a schematic perspective view showing the state in which a fixture for biological tissue according to an embodiment of the present invention is attached to a tensile testing machine that is not shown.
[Figure 2] Figure 2 is a schematic side view of the state shown in Figure 1.
[Figure 3] Figure 3 is a schematic perspective view of the fixture.
[Figure 4] Figure 4 is a schematic side view of the fixture.
[Figure 5] Figure 5(A) is a schematic perspective view of the fixture with the posture of a holder changed from the posture in Figure 3 and viewed in a direction different from the viewing direction in Figure 3, and Figure 5(B) is a schematic perspective view of the fixture with the posture of the holder changed from the postures in Figures 3 and 4 and viewed in the same direction as the viewing direction in Figure 3.
[Figure 6] Figure 6(A) is a schematic longitudinal cross-sectional view of an accommodating body, and Figure 6(B) is a schematic bottom view of the accommodating body.
[Figure 7] Figure 7 is a schematic perspective view of a restricting block.
[Figure 8] Figure 8(A) is a schematic front view of an angle adjusting plate, and Figure 8(B) is a schematic cross-sectional view of the angle adjusting plate taken in the direction along the line A-A in Figure 8(A).

### Description of Embodiments

An embodiment of the present invention will be described below with reference to the drawings.

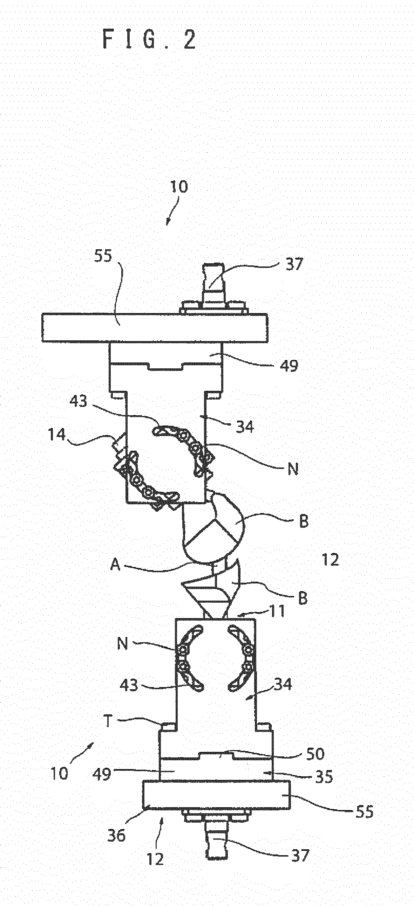

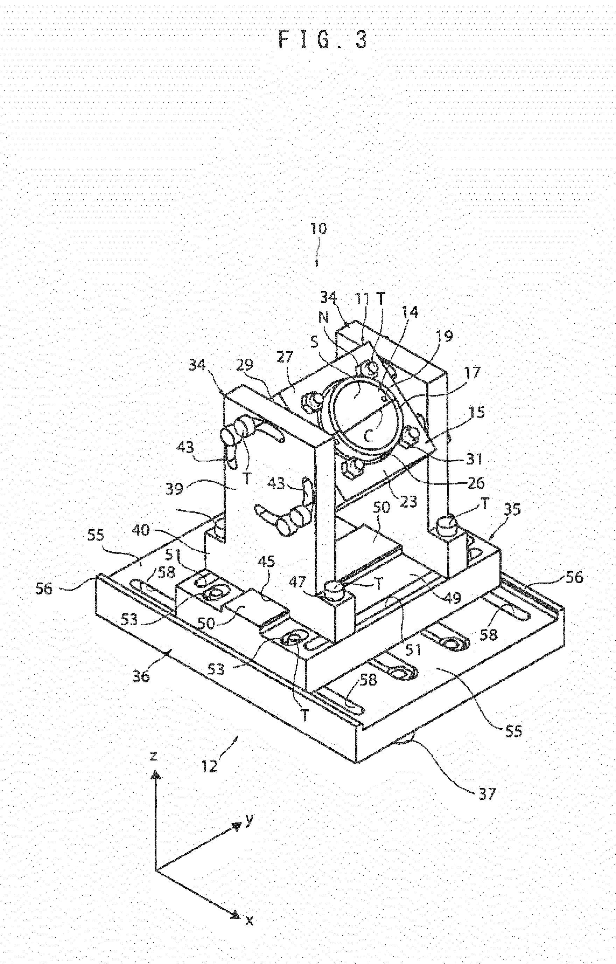

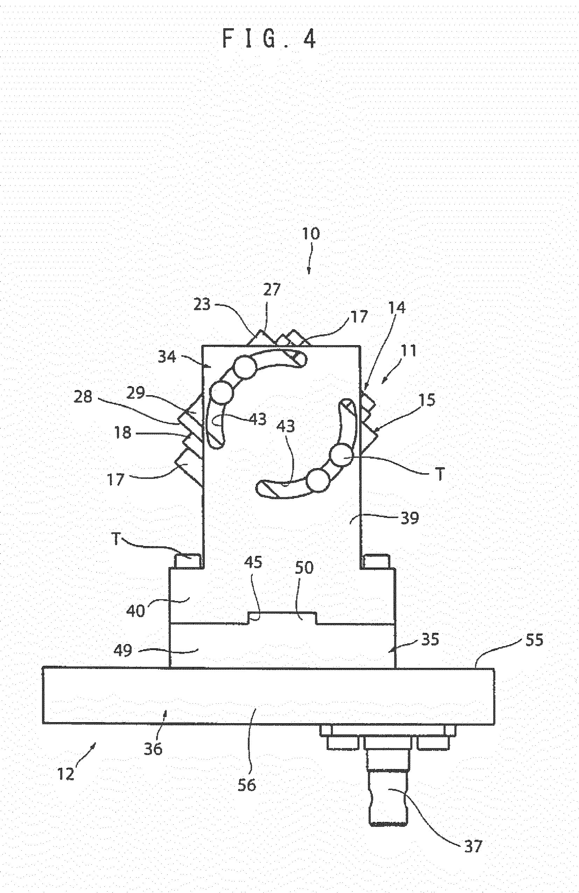

Figure 1 is a schematic perspective view showing the state in which a fixture for biological tissue according to the present embodiment is attached to a tensile testing machine that is not shown, and Figure 2 is a schematic side view of the state shown in Figure 1. Figure 3 is a schematic perspective view of the fixture, and Figure 4 is a schematic side view of the fixture. In these figures, the fixture 10 is used in a situation in which an artificial ligament A, which serves as biological tissue, is transplanted as the anterior cruciate ligament in the knee joint of an animal and the tensile testing machine exerts, after a predetermined period elapses, tensile force to the artificial ligament A in a knee joint portion taken out from the animal, and the fixture 10 is a jig for attaching the knee joint as a whole to the tensile testing machine. The fixture 10 according to the present embodiment is not necessarily made of a specific material and is made of a metal, such as stainless steel.

Two separate fixtures 10 having substantially the same structure are used when attached to the tensile testing machine. The two fixtures 10 fix and hold a thigh bone B as a first bone and a shinbone B as a second bone, respectively, and are attached to the tensile testing machine, as shown in Figures 1 and 2. Since the fixtures 10, which fix the thigh bone B and the shinbone B, have substantially the same structure, the thigh bone B and the shinbone B are collectively referred to as a "bone B" in the following description. Further, in the following sections, only the lower fixture 10 in Figures 1 and 2 will be described in terms of the configuration, effect, and other factors, and as for the upper fixture 10 in Figures 1 and 2, the configured portions identical or equivalent to those of the lower fixture 10 in Figures 1 and 2 have the same reference characters and will not described or will be described in a simplified manner.

The fixture 10 includes a holder 11, which holds the bone B with the artificial ligament A attached thereto, and a support 12, which supports the holder 11.

The holder 11 includes an accommodating body 14, which accommodates the side facing a base portion of the bone B, the side opposite the artificial ligament A, and a restricting block 15, which surrounds and holds the accommodating body 14.

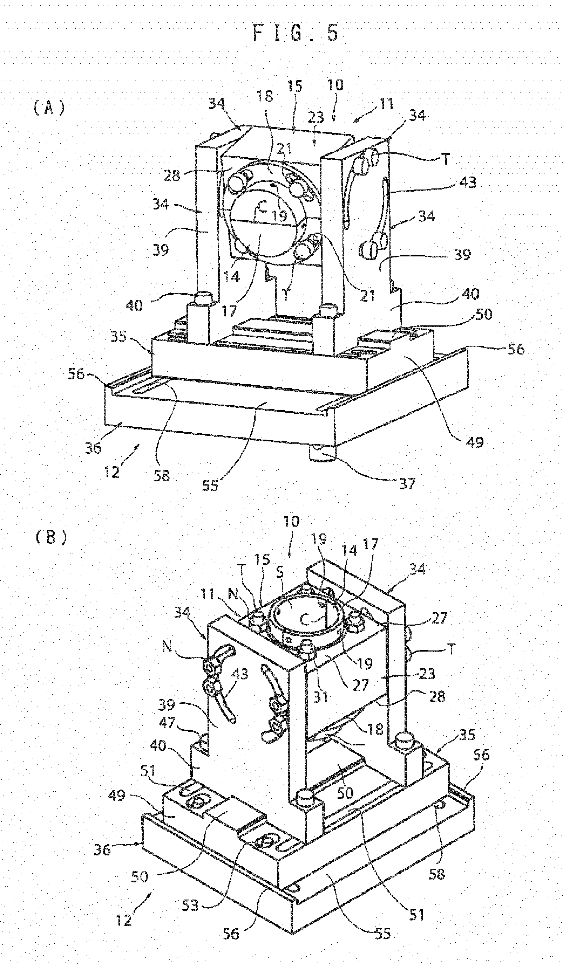

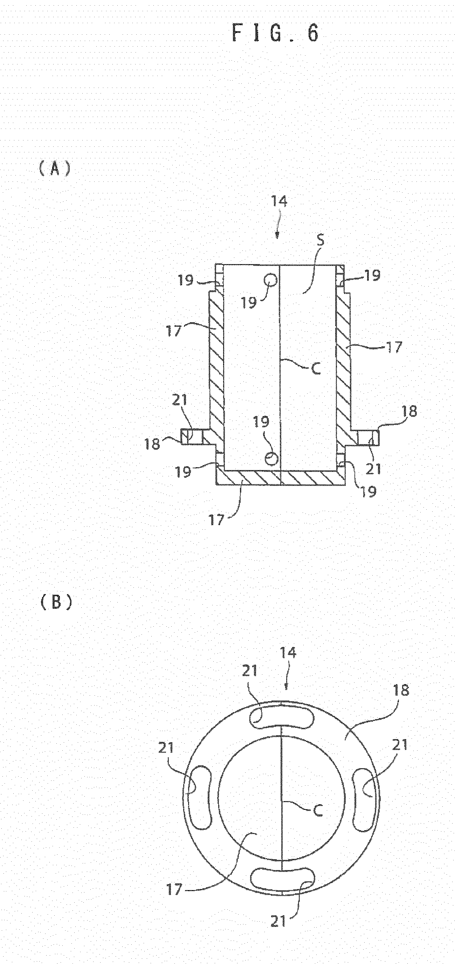

As shown in Figures 3 to 6, the accommodating body 14 is formed of a cylindrical member 17, which has an internal space S, into which the bone B is inserted, and a flange member 18, which is integrally continuous with the outer circumferential surface of the cylindrical member 17 (see Figures 5(A) and 6).

The cylindrical member 17 has one open end, which is the upper end in Figure 6(A), and a closed other end, which is the lower end in Figure 6(A) and forms a bottom, and can be divided into two halves along a joining edge C, which extends in the upward/downward direction in Figure 6(A). A plurality of holes 19, which pass through the cylindrical member 17 from the inner surface to the outer surface thereof, are formed in the outer circumferential surface of the cylindrical member 17.

The flange member 18 is provided around the cylindrical member 17 and in a position of the cylindrical member 17 shifted downward in Figure 6(A) and so provided as to be divided into two halves along the same joining edge C of the cylindrical member 17, as shown in Figure 6(B). The accommodating body 14 as a whole can therefore be divided into two halves. Further, the flange member 18 has elongated holes 21 extending in the circumferential direction, which pass through the flange member 18 from the front surface to the rear surface thereof, at four locations at equal intervals along the circumferential direction.

The accommodating body 14 is used as follows: The base-side portion of the bone B is inserted into the internal space S through an open portion of the cylindrical member 17, the portion located at the upper end thereof in Figure 6(A); the gap between the bone B and the cylindrical member 17 is then filled with a filler, such as an epoxy resin, so that the bone B is immobile in the internal space S; and screws that are not shown are inserted into the holes 19 to fix the bone B in a desired posture to the accommodating body 14. At this point, the bone B is so fixed that the bone axis thereof, which is the center axis of the bone B, roughly coincides with the center axis of the cylindrical member 17. After a test conducted by a tensile testing machine is completed, the screws are detached, the accommodating body 14 is divided, the epoxy resin is removed, and the bone B is detached from the accommodating body 14.

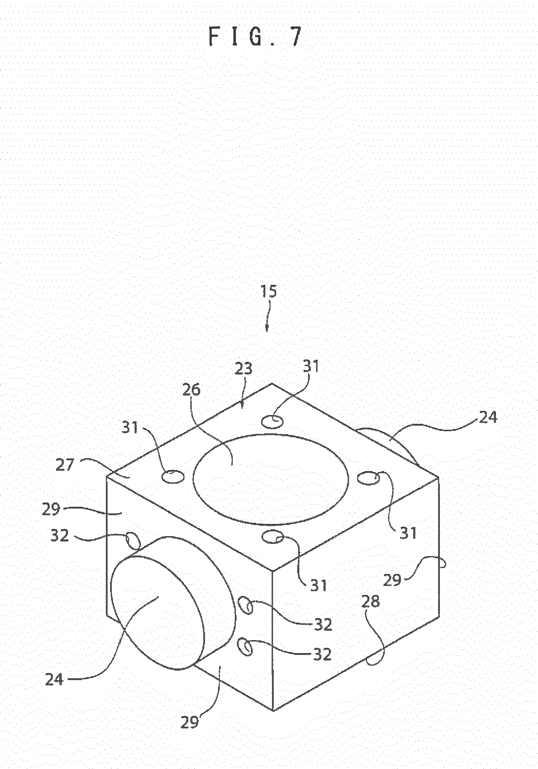

The restricting block 15 includes a main body 23, which has a box-like block shape, and protruding members 24, which are fixed to the main body 23 and each have a roughly columnar shape, as shown in Figure 7.

The main body 23 has a through hole 26, through which the cylindrical member 17 is inserted (see Figure 6 and other figures), a top surface 27 and a bottom surface 28, which form the opposite-end-side surfaces in the direction in which the through hole 26 extends, side surfaces 29, 29, which are continuous with the top surface 27 and the bottom surface 28 and to which the protruding members 24 are fixed, first positioning holes 31, which pass through the main body 23 between the top surface 27 and the bottom surface 28, and second positioning holes 32, which pass through the main body 23 between the side surfaces 29, 29.

The through hole 26 is a cylindrical hole that passes through the main body 23 between central portions of the top surface 27 and the bottom surface 28, has an inner diameter that is approximately equal to or slightly greater than the outer diameter of the cylindrical member 17, and is shorter than the cylindrical member 17.

When the cylindrical member 17 is inserted into the through hole 26 and attached to the main body 23, the flange member 18 comes into contact with the bottom surface 28, as shown in Figure 5(A), and the open-end side of the cylindrical member 17, the side opposite the flange member 18, is so disposed as to protrude beyond the top surface 27, as shown in Figure 5(B) and other figures.

The first positioning holes 31 are formed in the area outside the through hole 26 and at four locations at equal intervals along the circumferential direction, as shown in Figure 7. When the flange member 18 is attached to the main body 23, the first positioning holes 31 each communicate with part of the corresponding elongated hole 21 of the flange member 18 (see Figure 6(B) and other figures). The cylindrical member 17 inserted through the through hole 26 of the main body 23 is rotatable around the center axis of the cylindrical member 17 relative to the main body 23. The elongated holes 21, which communicate with the first positioning holes 31, are positioned while the cylindrical member 17 is rotated, and bolts T (see Figure 5(A)) are inserted through the positioned elongated holes 21 and fastened with nuts N (see Figure 5(B)), whereby the cylindrical member 17 is so fixed to the main body 23 as not to be rotatable. Therefore, rotating the cylindrical member 17 relative to the main body 23 allows the bone B accommodated in the cylindrical member 17 to be adjusted in terms of the angle of rotation around the bone axis of the bone B, and the cylindrical member 17 is fixed at a desired angle of rotation to the main body 23 with the bolts T and the nuts N in such a way that the cylindrical member 17 is not rotatable relative to the main body 23. The accommodating body 14 and the restricting block 15 thus form an around-bone-axis angle adjusting mechanism that adjusts the angle of rotation of the bone B around the bone axis thereof.

The second positioning holes 32 are formed at four locations on the side facing the outer edge of the side surfaces 29 and in positions where the second positioning holes 32 do not interfere with the through hole 26 and the first positioning holes 31, as shown in Figure 7.

The protruding members 24 are so provided as to protrude from central portions of the side surfaces 29, 29, the portions inside the second positioning holes 32.

As shown in Figures 3 and 4, the support 12 includes a pair of angle adjusting plates 34, 34, to which the holder 11 is so attached that the angle of rotation of the holder 11 is variable, a lateral direction adjusting plate 35, which supports the angle adjusting plates 34, 34 in a lateral movable manner along the y axis, a longitudinal direction adjusting plate 36, which supports the lateral direction adjusting plate 35 in a longitudinally movable manner along the x axis, and a chuck attaching tool 37, which is fixed to a surface of the longitudinal direction adjusting plate 36, the surface located on the lower side in Figure 3, and forms a portion attached to a chuck of the tensile testing machine.

The angle adjusting plates 34, 34, each of which has a roughly T-letter-like shape in a side view, are so disposed as to face each other and supportably sandwich the holder 11 and are each formed of a primary section 39, with which the restricting block 15 is in contact, and a bottom section 40, which is continuous with the one side of the primary section 39, the lower side in Figure 3, and is wider than the primary section 39.

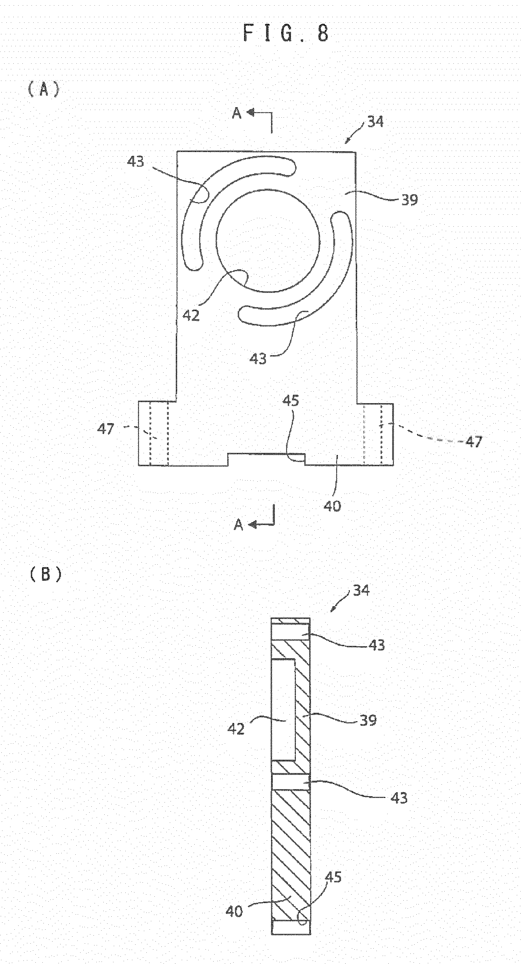

As shown in Figure 8, a circular recess 42 and elongated holes 43, 43 are formed in the primary section 39; the corresponding protruding member 24 (see Figure 7) of the restricting block 15 is rotatably fit into the circular recess 42, and the elongated holes 43, 43 are formed at two locations around the recess 42 and extend in the circumferential direction of a circle concentric with the recess 42. Figure 8(A) shows the angle adjusting plate 34 located on the right in Figure 3 and viewed from the side facing the inner surface thereof.

The recess 42 is so formed that the inner surface thereof facing the other angle adjusting plate 34 is open and so configured that when the protruding members 24, 24 are fit into the recesses 42, 42, the restricting block 15 is placed across the space between the angle adjusting plates 34, 34.

The elongated holes 43 are so formed as to pass through the primary section 39 and formed in positions where part of the elongated holes 43 communicate with the second positioning holes 32 (see Figure 7) of the restricting block 15 when the protruding members 24 fit into the recesses 42 are rotated. The elongated holes 43, 43 formed in the angle adjusting plates 34, 34 are formed in positions facing each other.

Achieve now the state in which the restricting block 15 to which the accommodating body 14 is attached is disposed between the pair of angle adjusting plates 34, 34, the protruding members 24, 24 are fit into the recesses 42, 42, the restricting block 15 is rotated with the protruding members 24, 24 serving as pivotal points, and a desired angle of rotation of the restricting block 15 is achieved. The bolts T are then inserted through the elongated holes 43, 43 from the exterior of one of the angle adjusting plates 34 to a point where the front ends of the bolts T protrude through the elongated holes 43, 43 of the other angle adjusting plate 34 on the opposite side, and the front ends of the bolts T are fastened with nuts N, whereby the holder 11 is so attached to and between the angle adjusting plates 34, 34 as not to be rotatable. The restricting block 15 can therefore be fixed to the angle adjusting plates 34, 34 after the restricting block 15 is rotated around the y axis and positioned, and the holder 11 can be attached to the angle adjusting plates 34, 34 in a variety of postures in which the angle of rotation of the holder 11 around the y axis has different values, as shown in Figures 3 and 5. The restricting block 15 and the angle adjusting plates 34, 34 thus form a bone inclination angle adjusting mechanism that adjusts the inclination angle of the bone B held by the holder 11.

A cutout 45 and third positioning holes 47, 47 are formed in the bottom section 40; the cutout 45 is open downward at the center on one side of the bottom section 40, the lower end side in Figure 8(A), and third positioning holes 47, 47 are formed in outwardly sticking out portions of the primary section 39, right and left side portions thereof in Figure 8(A), and pass through the portions in the upward/downward direction in Figure 8 (A).

As shown in Figure 3, the lateral direction adjusting plate 35 includes a base 49, which has a rectangular shape in a plan view, and a guide 50, which is located at the center of the base 49, protrudes from the upper surface of the base 49, and extends in the lengthwise direction thereof and with which the cutouts 45 of the angle adjusting plates 34 engage.

Elongated holes 51, 51 and fourth positioning holes 53 are formed in the base 49; the elongated holes 51, 51 are positioned on opposite sides in the widthwise direction of the base 49, extend in the lengthwise direction thereof, and pass through the base 49 in the upward/downward direction in Figure 3, and the fourth positioning holes 53 pass through the base 49 at four locations close to the corners thereof.

The elongated holes 51, 51 are formed in positions where part of the elongated holes 51, 51 communicate with the third positioning holes 47, 47 in a state in which the cutouts 45 are fit to the guide 50 and the angle adjusting plates 34 are so disposed as to stand on the base 49.

Achieve now a state in which the pair of angle adjusting plates 34, 34 are so disposed as to stand on the base 49, and the angle adjusting plates 34, 34 are moved in the lateral direction (y-axis direction) along the guide 50 and reach a desired lateral position. Bolts T are then inserted through the third positioning holes 47, 47 of the angle adjusting plates 34 to a point where the bolts T reach the elongated holes 51 of the base 49, and nuts N are inserted into the elongated holes 51 from the rear side of the base 49 to fasten the bolts T, whereby the angle adjusting plates 34 are so attached to the lateral direction adjusting plate 35 as not to be movable in the lateral direction. The holder 11 fixed to the angle adjusting plates 34, 34 can therefore be fixed to the lateral direction adjusting plate 35 after the position of the holder 11 in the lateral direction is adjusted. The angle adjusting plates 34, 34 and the lateral direction adjusting plate 35 thus form a lateral position adjusting mechanism that adjusts the lateral position of the holder 11 that holds the bone B.

The longitudinal direction adjusting plate 36 includes a base 55, which has a rectangular shape in a plan view, and guides 56, 56, which protrude upward in Figure 3 from edges of the base 55 on opposite sides in the widthwise direction thereof.

Elongated holes 58, 58 are formed in the base 55 and in the vicinity of the inner sides of the guide 56, 56, extend in the lengthwise direction thereof along the guides 56, 56, and pass through the base 55 in the upward/downward direction in Figure 3.

The guides 56, 56 are so disposed as to separate from each other by a distance approximately equal to or slightly wider than the lengthwise width of the base 49 of the lateral direction adjusting plate 35, and the lateral direction adjusting plate 35 is placed on an upper surface portion between the guides 56, 56.

The elongated holes 58, 58 are formed in positions where part of the elongated holes 58, 58 communicate with the fourth positioning holes 53, 53 of the lateral direction adjusting plate 35 with the lateral direction adjusting plate 35 placed on the upper surface of the longitudinal direction adjusting plate 36.

Achieve now a state in which the lateral direction adjusting plate 35 placed on the longitudinal direction adjusting plate 36 is moved in the longitudinal direction (x-axis direction) along the guides 56, 56 and reaches a desired longitudinal position. Bolts T are then inserted through the elongated holes 58, 58 from the rear side of the base 55 to a point where the bolts T reach the fourth positioning holes 53 of the lateral direction adjusting plate 35, and nuts N are inserted into the fourth positioning holes 53 to fasten front end portions of the bolts T, whereby the lateral direction adjusting plate 35 is so attached to the longitudinal direction adjusting plate 36 as not to be movable in the longitudinal direction. The lateral direction adjusting plate 35, which supports the angle adjusting plates 34, 34, to which the holder 11 is attached, can therefore be fixed to the longitudinal direction adjusting plate 36 after the longitudinal position of the lateral direction adjusting plate 35 is adjusted. The lateral direction adjusting plate 35 and the longitudinal direction adjusting plate 36 thus form a longitudinal position adjusting mechanism that adjusts the longitudinal position of the holder 11 that holds the bone B.

The chuck attaching tool 37 is fixed to the rear side of the base 55, the side opposite the direction in which the guides 56 protrude.

How to set the fixtures 10 in the tensile testing machine will next be described.

The artificial ligament A is transplanted in the knee joint of an animal, and the animal is forced to move for a predetermined period. After the animal is killed as a sacrifice, the knee joint, which is a target of an experiment, is isolated as a whole. With the artificial ligament A located between the thigh bone B and the shinbone B and linked thereto, the thigh bone B and the shinbone B are held by the two fixtures 10, and the chuck attaching tools 37, 37 are used to attach one of the fixtures 10 to the fixed side of the tensile testing machine and attach the other fixture 10 to the load cell side of the tensile testing machine.

To conduct a test for the stability of the isolated knee joint, the bone inclination angle adjusting mechanism is used to change the inclination angle of the thigh bone B and the shinbone B and adjust the bending angle of the knee joint, which is the angle between the thigh bone B and the shinbone B. With the bending angle changed several times, the tensile testing machine exerts several loads to the artificial ligament A, and the amounts of movement of the shinbone B relative to the thigh bone B are measured.

When the artificial ligament A in the isolated knee joint is pulled in a tensile test, to achieve a fixed posture of the artificial ligament A, which is a target of the test, the positions and postures of the thigh bone B and the shinbone B are adjusted as follows when the thigh bone B and the shinbone B are fixed by the fixtures 10: That is, the angle of rotation of the bone B around the bone axis thereof is so adjusted by the angle adjusting mechanism that a thigh bone B and a shinbone B are held by the holders 11, 11 with twist of the artificial ligament A eliminated or the amount of twist of the artificial ligament A fixed. Further, the thigh bone and the shinbone B are so held by the holders 11, 11 that adjustment of the horizontal position of the holders 11 performed by lateral position adjusting mechanism and the longitudinal position adjusting mechanism and adjustment of the inclination angles of the thigh bone B and the shinbone B performed by the bone inclination angle adjusting mechanism allow the artificial ligament A to be disposed in the vertical direction along the tensile direction of the tensile testing machine (Z-axis direction in Figure 1).

Therefore, according to the embodiment described above, the bone B can be held by the fixtures 10 with two degrees of rotation freedom and two degrees of translation freedom. In addition to the degrees of freedom described above, another degree of freedom in the tensile direction of the tensile testing machine advantageously allows the thigh bone B and the shinbone B to be held with five degrees of freedom, which are equivalent to motion of the knee joint formed of the thigh bone B and the shinbone B.

The fixture 10 in the present invention is not necessarily used to hold the knee joint as in the embodiment described above and is capable of holding a bone in another site with another ligament, tendon, or any other biological tissue attached to the bone, and the bone with the biological tissue attached thereto can be attached to the tensile testing machine.

In addition to the above, the configuration of each portion of the fixture in the present invention is not limited to the illustrated exemplary configuration and can be changed in a variety of manners to the extent that substantially the same effect is provided.

### Industrial Applicability

The fixture according to the present invention can be used as a jig for a test that holds a bone with a ligament, tendon, or any other biological tissue attached thereto and allows a tensile test of the biological tissue maintained in a desired posture.

### Reference Signs List

10 Fixture
11 Holder
12 Support
14 Accommodating body (around-bone-axis angle adjusting mechanism)
15 Restricting block (around-bone-axis angle adjusting mechanism, bone inclination angle adjusting mechanism)
34 Angle adjusting plate (bone inclination angle adjusting mechanism, lateral position adjusting mechanism)
35 Lateral direction adjusting plate (lateral position adjusting mechanism, longitudinal position adjusting mechanism)
36 Longitudinal direction adjusting plate (longitudinal position adjusting mechanism)

## Claims

1. A biological tissue fixture that is used when biological tissue connected to a bone is set on a tensile testing machine and fixes the bone with the biological tissue attached thereto, the fixture comprising:
a holder that holds the bone; and
a support that supports the holder,
wherein the holder is provided with an around-bone-axis angle adjusting mechanism that adjusts an angle of rotation of the bone around a bone axis of the bone held by the holder,
the holder and the support are provided with a bone inclination angle adjusting mechanism that adjusts an inclination angle of the bone held by the holder, and
the support is provided with a lateral position adjusting mechanism that adjusts a lateral position of the holder and a longitudinal position adjusting mechanism that adjusts a longitudinal position of the holder.

2. A fixture attaching method used when fixtures according to claim 1 are used to set a biological tissue connected to first and second bones on the tensile testing machine, the method comprising:
fixing the first bone with one of the fixtures and the second bone with another of the fixtures; and
attaching one of the fixtures to a fixed side of the tensile testing machine and attaching another of the fixtures to a load cell side of the tensile testing machine.

3. The fixture attaching method according to claim 2,
wherein when the first and second bones are fixed with the fixtures, the angle of rotation of each of the fixtures around the bone axis is so adjusted by the angle adjusting mechanism of each of the fixtures that the first and second bones are held by the holders with twist of the biological tissue eliminated or an amount of twist of the biological tissue fixed.

4. The fixture attaching method according to claim 2,
wherein when the first and second bones are fixed with the fixtures, a relative positional relationship between the holders in a horizontal plane is so adjusted by the lateral position adjusting mechanism and the longitudinal position adjusting mechanism of each of the fixtures that the first and second bones are held by the holders with the biological tissue disposed in a posture that accords with a tensile direction of the tensile testing machine.

5. The fixture attaching method according to claim 2,
wherein when the first and second bones are fixed with the fixtures, inclination angles of the first and second bones are so adjusted by the bone inclination angle adjusting mechanism of each of the fixtures that the first and second bones are held by the holders with a bending angle of each of the first and second bones being a desired value.
